(19) **Europäisches Patentamt**
**European Patent Office**
**Office européen des brevets**

(11) **EP 2 328 559 B1**

(12) **EUROPEAN PATENT SPECIFICATION**

(45) Date of publication and mention
of the grant of the patent:
**07.01.2015 Bulletin 2015/02**

(51) Int Cl.:
*A61K 9/19* (2006.01)       *A61K 9/08* (2006.01)
*A61K 39/395* (2006.01)       *C07K 16/28* (2006.01)

(21) Application number: **09782818.0**

(86) International application number:
**PCT/EP2009/061692**

(22) Date of filing: **09.09.2009**

(87) International publication number:
**WO 2010/031720 (25.03.2010 Gazette 2010/12)**

(54) **FORMULATION COMPRISING ANTIBODY AGAINST P-SELECTIN**

FORMULIERUNG VON ANTIKÖRPERN GEGEN P-SELECTIN

FORMULATION COMPRENANT UN ANTICORPS CONTRE P-SELECTIN

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **19.09.2008 EP 08164746**

(43) Date of publication of application:
**08.06.2011 Bulletin 2011/23**

(73) Proprietor: **F. Hoffmann-La Roche AG
4070 Basel (CH)**

(72) Inventors:
• **GRAUSCHOPF, Ulla
CH-4125 Riehen (CH)**
• **MAHLER, Hanns-Christian
CH-4001 Basel (CH)**
• **STAUCH, Oliver, Boris
79102 Freiburg (DE)**

(74) Representative: **Klostermeyer-Rauber, Dörte
F. Hoffmann-La Roche AG
Corporate Law Patents (CLP)
Grenzacherstrasse 124
4070 Basel (CH)**

(56) References cited:
**WO-A1-93/21956       WO-A1-98/56418
WO-A2-2007/109221       US-A- 5 800 815
US-A1- 2003 138 417**

• **DATABASE EMBASE [Online] ELSEVIER
SCIENCE PUBLISHERS, AMSTERDAM, NL
January 2007 WANG W ET AL: 'Antibody
structure, instability, and formulation' Database
accession no. EMB-2007029167**
• **WANG W ET AL: "Antibody structure, instability,
and formulation", January 2007 (2007-01),
JOURNAL OF PHARMACEUTICAL SCIENCES
200701 US, VOL. 96, NR. 1, PAGE(S) 1 - 26 ISSN:
0022-3549**

Remarks:
The file contains technical information submitted after
the application was filed and not included in this
specification

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

## Description

The present invention relates to a pharmaceutical formulation according to claim 1 of an antibody against P-Selectin and uses of the formulation.

The formulation according to the invention can be in a liquid form, a lyophilized form or in a liquid form reconstituted from a lyophilized form.

Antibodies against P-Selectin are known from, e.g., US patent 4,783,399, WO 93/06863, Geng et al (J. Biol. Chem., 266 (1991) 22313-22318), WO 93/21956 and WO 2005/100402. US 2003/0138417 discloses a pharmaceutical formulation comprising 50 and 100 mg/ml of the anti-L-selectin antibody HuEP5C7 in 50 mM histidine buffer, 125 mM sodium chloride and 0.01% Tween 80 at pH 6.0.

Exemplary antibodies against P-Selectin are described in WO 2005/100402 and include antibodies which are characterized in that the variable heavy chain amino acid sequence CDR3 of said antibody is selected from the group consisting of the heavy chain CDR3 sequences SEQ ID NO: 38, 39, 40, 41 or 42.

The antibodies are characterized in that said antibody binds P-selectin and comprises a variable heavy and light region selected from the group consisting of the heavy chain variable domain defined by amino acid sequence SEQ ID NO:4 and the light chain variable domain defined by SEQ ID NO:3.

The CDR sequences can be determined according to the standard definition of Kabat et al., Sequences of Proteins of Immunological Interest, 5th ed., Public Health Service, National Institutes of Health, Bethesda, MD (1991). CDRs on each chain are separated by framework amino acids. CDRs of SEQ ID NO: 1-22 are shown in SEQ ID NO: 29-52.

In one embodiment, the antibody is characterized in binding P-selectin and does not bind complement factor C1q and/ or Fc receptor. These antibodies do not elicit the complement dependent cytotoxicity (CDC) and/or antibody-dependent cellular cytotoxicity (ADCC). Preferably, this antibody is characterized in that it binds P-selectin, contains a Fc part derived from human origin and does not bind complement factor C1q. More preferably, this antibody is a human or humanized antibody.

In another embodiment, the antibody is characterized in that the constant chains are of human origin. Such constant chains are well known in the state of the art and e.g. described by Kabat (see e.g. Johnson and Wu, Nucleic Acids Res. 28 (2000) 214-218). For example a useful human heavy chain constant region comprises an amino acid sequence independently selected from the group consisting of SEQ ID NO: 24, 25, 26, 27 and 28. For example an useful human light chain constant region comprises an amino acid sequence of a kappa-light chain constant region of SEQ ID NO: 23.

The term "binding to P-selectin" as used herein means the binding of the antibody to P-selectin in either a BIAcore assay (Pharmacia Biosensor AB, Uppsala, Sweden) or in an ELISA in which either purified P-selectin or P-selectin CHO transfectants are coated onto microtiter plates.

In the BIAcore assay, the antibody is bound to a surface and binding of P-selectin is measured by Surface Plasmon Resonance (SPR). The affinity of the binding is defined by the terms ka (rate constant for the association of the antibody from the antibody/antigen complex), kd (dissociation constant), and $K_D$ (kd/ka). In further another embodiment, the antibodies show a $K_D$ of $10^{-8}$ or less, preferably of about $10^{-11}$ to $10^{-9}$ M (see examples). Accordingly, preferred antibody is that as decribed above, wherein the antibody bind to P-selectin with a $K_D$ value of less than $10^{-8}$ M in a BIAcore assay, preferably wherein the $K_D$ range is $10^{-11}$ to $10^{-9}$ M.

Preferably, the antibody is of IgG1 or IgG4 human subtype.

More preferably, the antibody is characterized in that the antibody is an antibody of human subclass IgG1, containing at least one mutation in L234, L235, D270, N297, E318, K320, K322, P331 and/or P329 or an antibody of human subclass IgG4, containing at least one mutation in L235 and S228 (numbering according to EU index).

In the P-selectin-specific ELISA purified P-selectin is coated onto microtiter plates and the binding of the antibody to P-selectin is detected with a biotinylated anti-human IgG and the usual steps of an ELISA. The $EC_{50}$ values in this assay range preferably between 0.002 and 0.03 $\mu$g/ml on P-selectin CHO cells, i.e. preferred antibodies are those, wherein the EC50 values for P-selectin binding are in the range of 0.002 to 0.03 $\mu$g/ml on P-selectin presenting CHO cells in an ELISA assay. In an assay in which P-selectin expressing CHO transfectants are coated onto the microtiter plate, the EC50 values range between 0.01 and 0.08 $\mu$g/ml, preferably between 0.01 and 0.04 $\mu$g/ml.

$EC_{50}$ values on E- and L-selectin transfectants are preferably above 100 $\mu$g/ml. The preferred antibodies are characterized in that they bind at least 1000 fold more specifically to P-selectin than to E- and/or L-selectin as measured by $EC_{50}$ values in an ELISA assay, wherein P- and E- and/or L-selectin are coated onto the microtiter plate.

The antibodies are preferably capable of binding to P-selectin in the presence of the P-selectin fragment aa 60-75 (Swiss-Prot sequence P16109) and/or do not competitively inhibit the binding of an antibody secreted by a cell line designated ATCC Accession No. HB11041 to P-selectin.

The antibodies preferably do not inhibit the interaction of P-selectin with platelet membrane glycoprotein GPIb$\alpha$ in an ELISA assay format. In the ELISA glycocalicin, the soluble extracellular portion of GPIb$\alpha$ was immobilized on the wells of microtiter plates, as described (Romo et al., J Exp Med 190:803 (1999), and the binding of purified P-selectin after preincubation with the P-selectin HuMabs was detected with a polyclonal anti-P-selectin antibody.

The preferred antibody is characterized in that it does not bind the C3 protein, more preferably it is characterized in that it does not elicit complement-dependent cytotoxicity (CDC). Further, the antibody may be characterized it does not bind to Fcγ receptors on NK effector cells. Preferably, the antibody is characaterized that it is an antibody of human subclass IgG1, containing at least one mutation in L234, L235, D270, N297, E318, K320, K322, P331 and/or P329 or an antibody of human subclass IgG4, containing at least one mutation in L235 and S228 (numbering according to EU index). The preferred antibody is characterized in that it does not elicit antibody-dependent cellular cytotoxicity (ADCC).

The antibodies comprise the light chain variable domain defined by amino acid sequence SEQ ID NO:3 and the heavy chain variable domain defined by SEQ ID NO:4.

The preferred antibodies are characterized in that the antibodies are of human IgG4 subclass or comprise at least one amino acid mutation causing non-binding to complement factor C1q. These variant antibodies comprise for example the amino acid sequence independently selected from the group consisting of SEQ ID NO: 25 or SEQ ID NO:26 and SEQ ID NO:28.

A "variant" anti-P-selectin antibody, refers herein to a molecule which differs in amino acid sequence from a "parent" anti-P-selectin antibody amino acid sequence by virtue of addition, deletion and/or substitution of one or more amino acid residue(s) in the parent antibody sequence. Preferably, the variant comprises one or more amino acid substitution(s) in one or more constant or variable region(s) of the parent antibody, more preferably in the constant region. For example, the variant may comprise at least one, e.g. from about one to about ten, and preferably from about two to about five, substitutions in one or more variable regions of the parent antibody. Ordinarily, the variant will have an amino acid sequence having at least 90% amino acid sequence identity with the parent antibody constant and/or variable domain sequences, more preferably at least 95%, and most preferably at least 99%.

Identity or homology with respect to this sequence is defined herein as the percentage of amino acid residues in the candidate sequence that are identical with the parent antibody residues, after aligning the sequences and introducing gaps, if necessary, to achieve the maximum percent sequence identity. None of N-terminal, C-terminal, or internal extensions, deletions, or insertions into the antibody sequence shall be construed as affecting sequence identity or homology. The variant retains the ability to bind human P-selectin and preferably has properties, which are superior to those of the parent antibody. For example, the variant may have a stronger binding affinity, enhanced ability to treat a disease associated with critical limb ischemia or peripheral arterial occlusive disease (CLI/PAOD).

The variant antibody of particular interest herein is one which displays at least about 4 fold, enhancement in inhibitory activity in the adhesion assay when compared to the parent antibody because of the elimination of the binding to the Fcγ receptors.

The "parent" antibody herein is one, which is encoded by an amino acid sequence used for the preparation of the variant. Preferably, the parent antibody has a human framework region and, if present, has human antibody constant region(s). For example, the parent antibody may be a humanized or human antibody.

The antibodies according to the invention include, in addition, such antibodies having "conservative sequence modifications", nucleotide and amino acid sequence modifications, which do not affect or alter the above-mentioned characteristics of the antibody according to the invention. Modifications can be introduced by standard techniques known in the art, such as site-directed mutagenesis and PCR-mediated mutagenesis. Conservative amino acid substitutions include ones in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g. glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine). Thus, a predicted nonessential amino acid residue in a human anti-P-selectin antibody can be preferably replaced with another amino acid residue from the same side chain family.

Amino acid substitutions can be performed by mutagenesis based upon molecular modeling as described by Riechmann et al., Nature 332 (1988) 323-327 and Queen et al., Proc. Natl. Acad. Sci. USA 86 (1989) 10029-10033.

Further preferred antibodies comprise an κ-light chain constant region as defined by SEQ ID NO:23.

Further preferrd antibodies are those defined as IgG1v1 (PVA-236; GLPSS331 as specified by E233P; L234V; L235A; delta G236; A327G; A330S; P331S), IgG1v2 (L234A; L235A) and IgG4v1 (S228P; L235E).


Description of the sequence listing


SEQ ID NO:1        LC1004-001 light chain, variable domain of HuMab 1004-001
SEQ ID NO:2        LC1004-001 heavy chain, variable domain of HuMab 1004-001

SEQ ID NO:3 LC 1004-002 light chain, variable domain of HuMab 002
SEQ ID NO:4 LC 1004-002 heavy chain, variable domain of HuMab 002
SEQ ID NO:5 LC 1004-003 light chain, variable domain of HuMab 003
SEQ ID NO:6 LC 1004-003 heavy chain, variable domain of HuMab 003
SEQ ID NO:7 LC 1004-004 light chain (I), variable domain of HuMab 004 (I)
SEQ ID NO:8 LC 1004-004 heavy chain (I), variable domain of HuMab 004 (I)
SEQ ID NO:9 LC 1004-004 light chain (II), variable domain of HuMab 004 (II)
SEQ ID NO:10 LC 1004-004 heavy chain (II), variable domain of HuMab 004 (II)
SEQ ID NO:11 Light chain, variable domain of HuMab 005
SEQ ID NO:12 Heavy chain, variable domain of HuMab 005
SEQ ID NO:13 Light chain, variable domain of HuMab 010 (I)
SEQ ID NO:14 Heavy chain, variable domain of HuMab 010 (I)
SEQ ID NO:15 Light chain, variable domain of HuMab 010 (II)
SEQ ID NO:16 Heavy chain, variable domain of HuMab 010 (II)
SEQ ID NO:17 Light chain, variable domain of HuMab 010 (III)
SEQ ID NO:18 Heavy chain, variable domain of HuMab 010 (III)
SEQ ID NO:19 Light chain,variable domain of HuMab 011
SEQ ID NO:20 Heavy chain, variable domain of HuMab 011
SEQ ID NO:21 Light chain, variable domain of HuMab 017
SEQ ID NO:22 Heavy chain, variable domain of HuMab 017
SEQ ID NO:23 $\kappa$ light chain constant region
SEQ ID NO:24 $\gamma$1 heavy chain constant region
SEQ ID NO:25 $\gamma$1 heavy chain constant region PVA236/GLPSS331 (IgG1v1)
SEQ ID NO:26 $\gamma$1 heavy chain constant region L234A/L235A (IgG1v2)
SEQ ID NO:27 $\gamma$4 heavy chain constant region
SEQ ID NO:28 $\gamma$4 heavy chain constant region S228/L235E (IgG4v1)
SEQ ID NO:29-32 Heavy chain CDR1
SEQ ID NO:33-37 Heavy chain CDR2
SEQ ID NO:38-42 Heavy chain CDR3
SEQ ID NO:43-44 Light chain CDR1
SEQ ID NO:45-46 Light chain CDR2
SEQ ID NO:47-52 Light chain CDR3

In one embodiment the present invention provides a formulation wherein the antibody is present in an amount in the range of from 1 to 50 mg/mL. The antagonistic monoclonal antibodies against P-selectin may be produced by hybridoma cell lines. The preferred hybridoma cell lines are hu-Mab<P-selectin>LC 1004-001 (antibody HuMab 001) hu-Mab<P-selectin>LC 1004-002 (antibody HuMab 002) and hu-Mab<P-selectin>LC 1004-017 (antibody HuMab 017), which were deposited, under the Budapest Treaty on the international recognition of the deposit of microorganisms for the purposes of patent procedure, with Deutsche Sammlung von Mikroorganismen und Zellkulturen GmbH (DSMZ), Germany:

| Cell line | Deposition No. | Date of Deposit |
|---|---|---|
| hu-Mab<P-selectin>LC 1004-001 | DSM ACC2640 | 30.03.2004 |
| hu-Mab<P-selectin>LC 1004-002 | DSM ACC2641 | 30.03.2004 |
| hu-Mab<P-selectin>LC 1004-017 | DSM ACC2642 | 30.03.2004 |

The antibodies useful in the formulations according to the invention are preferably produced by recombinant means, e.g. by those described in WO2006/072564. Such methods are widely known in the state of the art and comprise protein expression in prokaryotic and eukaryotic cells with subsequent isolation of the antibody polypeptide and usually purification to a pharmaceutically acceptable purity. For the protein expression, nucleic acids encoding light and heavy chains or fragments thereof are inserted into expression vectors by standard methods. Expression is performed in appropriate prokaryotic or eukaryotic host cells like CHO cells, NS0 cells, SP2/0 cells, HEK293 cells, COS cells, yeast, or E.coli cells, and the antibody is recovered from the cells (supernatant or cells after lysis) by standard techniques, including alkaline/SDS treatment, CsCl banding, column chromatography, agarose gel electrophoresis, and others well known in the art, e.g. as described in WO2006/072564.

The term "buffer" as used herein denotes a pharmaceutically acceptable excipient, which stabilizes the pH of a pharmaceutical preparation. Suitable buffers are well known in the art and can be found in the literature. Pharmaceutically acceptable buffers are histidine-buffers, citrate-buffers, succinate-buffers, acetate-buffers and phosphate-buffers. The buffer is L-histidine hydrochloride with pH adjustment with an acid or a base known in the art. This buffer is used in an amount of 20 mM. Independently from the buffer used, the pH is adjusted at a value of 6.0 with an acid or a base known in the art, e.g. hydrochloric acid, acetic acid, phosphoric acid, sulfuric acid and citric acid, sodium hydroxide and potassium hydroxide.

The term "surfactant" as used herein denotes a pharmaceutically acceptable excipient which is used to protect protein formulations against mechanical stresses like agitation and shearing. Examples of pharmaceutically acceptable surfactants include polyoxyethylensorbitan fatty acid esters (Tween), polyoxyethylene alkyl ethers (Brij), alkylphenylpolyoxyethylene ethers (Triton-X), polyoxyethylene-polyoxypropylene copolymer (Poloxamer, Pluronic), and sodium dodecyl sulphate (SDS). Polyoxyethylenesorbitan-fatty acid esters are polysorbate 20, (sold under the trademark Tween 20™) and polysorbate 80 (sold under the trademark Tween 80™). Preferred polyethylene-polypropylene copolymers are those sold under the names Pluronic® F68 or Poloxamer 188™. Polyoxyethylene alkyl ethers are those sold under the trademark Brij™. Alkylphenolpolyoxyethylene esthers are sold under the tradename Triton-X. Polysorbate 20 (Tween 20™) is present in a concentration of 0.2%.

The term "stabilizer" denotes a pharmaceutical acceptable excipient, which protects the active pharmaceutical ingredient and/or the formulation from chemical and/or physical degradation during manufacturing, storage and application. Chemical and physical degradation pathways of protein pharmaceuticals are reviewed by Cleland et al. (1993), Crit Rev Ther Drug Carrier Syst 10(4):307-77, Wang (1999) Int J Pharm 185(2):129-88, Wang (2000) Int J Pharm 203(1-2):1-60 and Chi et al. (2003) Pharm Res 20(9): 1325-36. Stabilizers include sugars, amino acids, polyols, cyclo-dextrines, e.g. hydroxypropyl-$\beta$-cyclodextrine, sulfobutylethyl-$\beta$-cyclodextrin, $\beta$-cyclodextrin, polyethylenglycols, e.g. PEG 3000, PEG 3350, PEG 4000, PEG 6000, albumine, human serum albumin (HSA), bovine serum albumin (BSA), salts, e.g. sodium chloride, magnesium chloride, calcium chloride, chelators, e.g. EDTA as hereafter defined. As mentioned hereinabove, stabilizers can be present in the formulation in an amount of about 10 to about 500 mM, preferably in an amount of about 10 to about 300 mM and more preferably in an amount of about 100 mM to about 300 mM.

The term "sugar" as used herein denotes a monosaccharide or an oligosaccharide. A monosaccharide is a monomeric carbohydrate which is not hydrolysable by acids, including simple sugars and their derivatives, e.g. aminosugars. Examples of monosaccharides include glucose, fructose, galactose, mannose, sorbose, ribose, deoxyribose, neuraminic acid. An oligosaccharide is a carbohydrate consisting of more than one monomeric saccharide unit connected via glycosidic bond(s) either branched or in a chain. The monomeric saccharide units within an oligosaccharide can be identical or different. Depending on the number of monomeric saccharide units the oligosaccharide is a di-, tri-, tetra-penta- and so forth saccharide. In contrast to polysaccharides the monosaccharides and oligosaccharides are water soluble. Examples of oligosaccharides include sucrose, trehalose, lactose, maltose and raffinose. Trehalose is present in an amount of 240mM.

The term "amino acid" as used herein denotes a pharmaceutically acceptable organic molecule possessing an amino moiety located at $\alpha$-position to a carboxylic group. Examples of amino acids include arginine, glycine, ornithine, lysine, histidine, glutamic acid, asparagic acid, isoleucine, leucine, alanine, phenylalanine, tyrosine, tryptophane, methionine, serine, proline. Amino acids are generally used in an amount of about 10 to 500 mM, preferably in an amount of about 10 to about 300 mM and more preferably in an amount of about 100 to about 300 mM.

The term "polyols" as used herein denotes pharmaceutically acceptable alcohols with more than one hydroxy group. Suitable polyols comprise to but are not limited to mannitol, sorbitol, glycerine, dextran, glycerol, arabitol, propylene glycol, polyethylene glycol, and combinations thereof Polyols can be used in an amount of about 10 mM to about 500 mM, preferably in an amount of about 10 to about 300 mM and more preferably in an amount of about 100 to about 300 mM.

A subgroup within the stabilizers are lyoprotectants. The term "lyoprotectant" denotes pharmaceutical acceptable excipients, which protect the labile active ingredient (e.g. a protein) against destabilizing conditions during the lyophilisation process, subsequent storage and reconstitution. Lyoprotectants comprise but are not limited to the group consisting of sugars, polyols (such as e.g. sugar alcohols) and amino acids. Lyoprotectants can be selected from the group consisting of sugars such as sucrose, trehalose, lactose, glucose, mannose, maltose, galactose, fructose, sorbose, raffinose, neuraminic acid, amino sugars such as glucosamine, galactosamine, N-methylglucosamine ("Meglumine"), polyols such as mannitol and sorbitol, and amino acids such as arginine and glycine or mixtures thereof. Lyoprotectants are generally used in an amount of about 10 to 500mM, preferably in an amount of about 10 to about 300 mM and more preferably in an amount of about 100 to about 300 mM.

A subgroup within the stabilizers are antioxidants. The term "antioxidant" denotes pharmaceutically acceptable excipients, which prevent oxidation of the active pharmaceutical ingredient. Antioxidants comprise but are not limited to ascorbic acid, gluthathion, cysteine, methionine, citric acid, EDTA. Antioxidants can be used in an amount of about 0.01 to about 100 mM, preferably in an amount of about 5 to about 50 mM and more preferably in an amount of about 5 to about 20 mM.

The term "tonicity agents" as used herein denotes pharmaceutically acceptable tonicity agents. Tonicity agents are used to modulate the tonicity of the formulation. The formulation can be hypotonic, isotonic or hypertonic. Isotonicity in general relates to the osmostic pressure relative of a solution usually relative to that of human blood serum. The formulation according to the invention can be hypotonic, isotonic or hypertonic but will preferably be isotonic. An isotonic formulation is liquid or liquid reconstituted from a solid form, e.g. from a lyophilised form and denotes a solution having the same tonicity as some other solution with which it is compared, such as physiologic salt solution and the blood serum. Suitable tonicity agents comprise but are not limited to sodium chloride, potassium chloride, glycerine and any component from the group of amino acids, sugars, in particular glucose. Tonicity agents are generally used in an amount of about 5 mM to about 500 mM.

Within the stabilizers and tonicity agents there is a group of compounds which can function in both ways, i.e. they can at the same time be a stabilizer and a tonicity agent. Examples thereof can be found in the group of sugars, amino acids, polyols, cyclodextrines, polyethyleneglycols and salts. An example for a sugar which can at the same time be a stabilizer and a tonicity agent is trehalose.

The compositions may also contain adjuvants such as preservatives, wetting agents, emulsifying agents and dispersing agents. Prevention of presence of microorganisms may be ensured both by sterilization procedures, and by the inclusion of various antibacterial and antifungal agents, for example, paraben, chlorobutanol, phenol, sorbic acid, and the like. Preservatives are generally used in an amount of about 0.001 to about 2 %(w/v). Preservatives comprise but are not limited to ethanol, benzyl alcohol, phenol, m-cresol, p-chlor-m-cresol, methyl or propyl parabens, benzalkonium chloride.

The term "liquid" as used herein in connection with the formulation according to the invention denotes a formulation which is liquid at a temperature of at least about 2 to about 8°C under atmospheric pressure.

The term "lyophilizate" as used herein in connection with the formulation according to the invention denotes a formulation which is manufactured by freeze-drying methods known in the art *per se.* The solvent (e.g. water) is removed by freezing following sublimation under vacuum and desorption of residual water at elevated temperature. The lyophilisate has usually a residual moisture of about 0.1 to 5% (w/w) and is present as a powder or a physical stable cake. The lyophilizate is characterized by a fast dissolution after addition of a reconstitution medium.

The term "reconstituted formulation" as used herein in connection with the formulation according to the invention denotes a formulation which is lyophilized and re-dissolved by addition of reconstitution medium. The reconstitution medium comprise but is not limited to water for injection (WFI), bacteriostatic water for injection (BWFI), sodium chloride solutions (e.g. 0.9% (w/v) NaCl), glucose solutions (e.g. 5% glucose), surfactant, containing solutions (e.g. 0.01% polysorbate 20), a pH -buffered solution (eg. phosphate-buffered solutions).

The formulations according to the invention have new and inventive properties causing a benefit for a patient suffering from asthma or an allergic disease.

The invention further comprises the use of a formulation according to the invention for the manufacture of a medicament for asthma treatment.

A composition of the present invention can be administered by a variety of methods known in the art. As will be appreciated by the skilled artisan, the route and/or mode of administration will vary depending upon the desired results.

To administer a composition of the invention by certain routes of administration, it may be necessary to dilute the composition in a diluent. Pharmaceutically acceptable diluents include saline, glucose, Ringer and aqueous buffer solutions.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion.

The composition must be sterile and fluid to the extent that the composition is deliverable by syringe. In addition to water, the carrier can be an isotonic buffered saline solution, ethanol, polyol (e.g., glycerol, propylene glycol, and liquid polyetheylene glycol, and the like), and suitable mixtures thereof

The formulation according to the invention can be administered by intravenous (i.v.), subcutaneous (s.c.) or any other parental administration means such as those known in the pharmaceutical art.

The formulation according to the invention can be prepared by methods known in the art, e.g. ultrafiltration-diafiltration, dialysis, addition and mixing, lyophilisation, reconstitution, and combinations thereof Examples of preparations of formulations according to the invention can be found hereinafter.

Examples

**Example 1: Preparation of liquid formulations**

huMAb-P-Selectin prepared and fermented and purified as described in WO 2005/100402 was provided at a concentration of approx. 22to 27 mg/mL in a 20 mM histidine buffer at a pH of approx. 6.0, or in a 20 mM acetate buffer at a pH of approx. 5.2.

For the preparation of the liquid formulations huMAb-P-Selectin the excipients (e.g. trehalose) were added as 2-10-fold stock solutions to the antibody solution. The surfactant was then added as a 2 to 200-fold stock solution or as pure surfactant. Finally the protein concentration was adjusted with a buffer to the final huMAb-P-Selectin concentration of approx. 15mg/mL.

All formulations were sterile-filtered through 0.22 $\mu$m low protein binding filters and aseptically filled under nitrogen atmosphere into sterile 6 mL glass vials closed with ETFE (Copolymer of ethylene and tetrafluoroethylene)-coated rubber stoppers and alucrimp caps. The fill volume was approximately 2.4 mL. These formulations were stored at different ICH climate conditions (5°C, 25°C and 40°C) for different intervals of time and stressed by shaking (1 week at a shaking frequency of 200 min$^{-1}$ at 5°C) and freeze-thaw stress methods. The samples were analyzed before and after applying the stress tests by the analytical methods 1) UV spectrophotometry, and 2) Size Exclusion Chromatography (SEC).

Size Exclusion Chromatography (SEC) was used to detect soluble high molecular weight species (aggregates) and low molecular weight hydrolysis products (LMW) in the formulations. The method was performed on a Water Alliance 2795 HPLC instrument equipped with a TOSOH BIOSCIENCE TSK G3000 SWXL column. Intact monomer, aggregates and hydrolysis products were separated by an isocratic elution profile, using 0.2M $K_2HPO_4$ /KOH, 0.25M KCL, pH 7.0 as mobile phase, and were detected at a wavelength of 280nm. UV spectroscopy, used for determination of protein content, was performed on a Varian Cary Bio UV spectrophotometer in a wavelength range from 240 nm to 400 nm. Neat protein samples were diluted to approx. 0.5 mg/mL with the corresponding formulation buffer. The protein concentration was calculated according to equation 1.

$$\text{Equation 1:} \quad \text{Protein content} = \frac{A(280) - A(320) \times dil.factor}{\varepsilon \left\langle cm^2 \middle/ mg \right\rangle \times d \left\langle cm \right\rangle}$$

The UV light absorption at 280 nm was corrected for light scattering at 320 nm and multiplied with the dilution factor, which was determined from the weighed masses and densities of the neat sample and the dilution buffer. The numerator was divided by the product of the cuvette's path length d and the extinction coefficient $\varepsilon$.

**Table 1:**

| Formulation A Storage at 2-8°C | | 15 mg/mL huMAb-P-Selectin, 20 mM L-histidine HCl, 240 mM trehalose, 0.02% polysorbate 20, at pH 6.0 | | |
|---|---|---|---|---|
| Timepoint | Protein conc. (mg/mL) | Size Exclusion - HPLC | | |
| | | HMW (%) | Monomer (%) | LMW (%) |
| Initial | 16.7 | 0.2 | 99.8 | 0.0 |
| 5 weeks | 15.8 | 0.2 | 99.8 | 0.0 |
| 11 weeks | 16.2 | 0.3 | 99.7 | 0.0 |
| 23 weeks | 16.6 | 0.4 | 99.0 | 0.6 |
| Formulation B (Reference) Storage at 2-8°C | | 15mg/mL huMAb-P-Selectin, 20 mM sodium acetate, 140 mM trehalose, | | |

(continued)

| Timepoint | Protein conc. (mg/mL) | Size Exclusion - HPLC | | |
|---|---|---|---|---|
| | | HMW (%) | Monomer (%) | LMW (%) |
| | | | 75 mM glycine 0.04% polysorbate 20, at pH 5.2 | |
| Timepoint | Protein conc. (mg/mL) | Size Exclusion - HPLC | | |
| | | HMW(%) | Monomer (%) | LMW (%) |
| Initial | 16.5 | 0.1 | 99.9 | 0.0 |
| 5 weeks | 16.1 | 0.2 | 99.8 | 0.0 |
| 11 weeks | 16.0 | 0.2 | 99.8 | 0.0 |
| 26 weeks | 15.6 | 0.1 | 99.9 | 0.0 |

**Example 2: Preparation of lyophilized formulations and liquid formulations reconstituted from lyophilized formulations**

huMAb-P-Selectin prepared and fermented and purified as described in WO 2005/100402 was provided at a concentration of approx. 22to 27 mg/mL in a 20 mM histidine buffer at a pH of approx. 6.0, or in a 20 mM succinate buffer at a pH of approx. 5.2, and lyophilized using the freeze-drying cycle reported in Table 2.

**Table 2 Freeze-drying Cycle type I**

| Step | Shelf temperature (°C) | Ramp Rate (°C/min) | Hold time (min) | Vacuum Set point ($\mu$bar) |
|---|---|---|---|---|
| Pre-cooling | 5°C | 0.0 | 60 | - |
| Freezing | -40°C | 1.0 | 120 | - |
| Primary Drying | -25°C | 0.5 | 3900 | 80 |
| Secondary Drying | +25°C | 0.2 | 300 | 80 |

The product was first cooled from room temperature to approx 5°C (pre-cooling), followed by a freezing step at -40°C with a plate cooling rate of approx. 1°C/min, followed by a holding step at -40°C for about 2 hours. The first drying step was performed at a plate temperature of approx. - 25°C and a chamber pressure of approx. 80 $\mu$bar for about 65 hours. Subsequently, the second drying step started with a temperature ramp of 0.2°C / min from -25°C to 25°C, followed by a holding step at 25°C for at least 5 hours at a chamber pressure of approx. 80 $\mu$bar.

Lyophilization was carried out in an Usifroid SMH-90 LN2 freeze-dryer (Usifroid, Maurepas, France). All lyophilized cakes had a residual water content of about 0.1 to 2.0% as determined by the Karl-Fischer method. The freeze-dried samples were incubated at different temperatures for different intervals of time.

The lyophilized formulations were reconstituted to a final volume of 5.3 mL with water for injection (WFI) yielding an isotonic formulation with an antibody concentration of approx. 15 mg/mL. The reconstitution time of the freeze-dried cakes was below 1 min. Analysis of the reconstituted samples was either performed immediately after reconstitution, or after a 24 hour incubation period of the reconstituted liquid sample at 25°C.

The samples were analyzed by 1) UV spectrophotometry and 2) Size Exclusion Chromatography (SEC).

**Table 3**

| | |
|---|---|
| **Formulation C** Storage at 2-8°C | 15 mg/mL huMAb-P-Selectin, 20 mM L-histidine HCl, 240 mM trehalose, 0.02% polysorbate 20, at pH 6.0 |

(continued)

| Timepoint | Protein conc. (mg/mL) | Size Exclusion - HPLC | | |
| --- | --- | --- | --- | --- |
| | | HMW (%) | Monomer (%) | LMW (%) |
| Initial | 16.7 | 0.2 | 99.8 | 0.0 |
| 5 weeks | 17.1 | 0.2 | 99.8 | 0.0 |
| 11 weeks | 17.0 | 0.2 | 99.8 | 0.0 |
| 23 weeks | 16.4 | 0.3 | 99.1 | 0.6 |
| **Formulation D Reference** Storage at 2-8°C | | 15 mg/mL huMAb-P-Selectin, 20 mM succinate, 240 mM trehalose, 20 mM arginine 0.02% polysorbate 20, at pH 6.0 | | |
| Timepoint | Protein conc. (mg/mL) | Size Exclusion - HPLC | | |
| | | HMW (%) | Monomer (%) | LMW (%) |
| Initial | 16.7 | 0.2 | 99.8 | 0.0 |
| 5 weeks | 17.0 | 0.2 | 99.8 | 0.0 |
| 11 weeks | 17.1 | 0.2 | 99.8 | 0.0 |
| 36 weeks | 16.5 | 0.2 | 99.8 | 0.0 |

SEQUENCE LISTING

<110> F. Hoffmann-La Roche AG

<120> NOVEL ANTIBODY FORMULATION

<130> 25380

<160> 28

<170> PatentIn version 3.2

<210> 1
<211> 107
<212> PRT
<213> Homo sapiens

<400> 1

```
Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
            35                  40                  45

Tyr Asp Ala Ser Asn Arg Ala Thr Gly Ile Pro Ala Arg Phe Ser Gly
        50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Asn Asn Trp Pro Leu
                85                  90                  95

Thr Phe Gly Gly Gly Thr Lys Val Glu Ile Lys
            100                 105
```

<210> 2
<211> 124
<212> PRT
<213> Homo sapiens

<400> 2

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30
```

10

Asp Met His Trp Val Arg Gln Ala Thr Gly Lys Gly Leu Glu Trp Val
35 40 45

Ser Gly Ile Thr Thr Ala Gly Asp Thr Tyr Tyr Pro Gly Ser Val Lys
50 55 60

Gly Arg Phe Thr Ile Ser Arg Glu Asn Ala Lys Asn Ser Leu Tyr Leu
65 70 75 80

Gln Met Asn Ser Leu Arg Ala Gly Asp Thr Ala Val Tyr Tyr Cys Ala
85 90 95

Arg Gly Arg Ile Ser Met Asp Arg Gly Val Lys Asn Asn Trp Phe Asp
100 105 110

Pro Trp Gly Gln Gly Thr Leu Val Thr Val Ser Ser
115 120

<210> 3
<211> 107
<212> PRT
<213> Homo sapiens

<400> 3

Glu Ile Val Leu Thr Gln Ser Pro Ala Thr Leu Ser Leu Ser Pro Gly
1 5 10 15

Glu Arg Ala Thr Leu Ser Cys Arg Ala Ser Gln Ser Val Ser Ser Tyr
20 25 30

Leu Ala Trp Tyr Gln Gln Lys Pro Gly Gln Ala Pro Arg Leu Leu Ile
35 40 45

Tyr Asp Ala Ser Asn Arg Ala Thr Gly Ile Pro Ala Arg Phe Ser Gly
50 55 60

Ser Gly Ser Gly Thr Asp Phe Thr Leu Thr Ile Ser Ser Leu Glu Pro
65 70 75 80

Glu Asp Phe Ala Val Tyr Tyr Cys Gln Gln Arg Ser Asn Trp Pro Leu
85 90 95

Thr Phe Gly Gly Gly Thr Lys Val Glu Ile Lys
100 105

<210> 4
<211> 124
<212> PRT

11

<213> Homo sapiens

<400> 4

```
        Glu Val Gln Leu Val Glu Ser Gly Gly Gly Leu Val Arg Pro Gly Gly
        1               5                   10                  15


        Ser Leu Arg Leu Ser Cys Ala Ala Ser Gly Phe Thr Phe Ser Asn Tyr
                    20                  25                  30


        Asp Met His Trp Val Arg Gln Ala Thr Gly Lys Gly Leu Glu Trp Val
                    35                  40                  45


        Ser Ala Ile Thr Ala Ala Gly Asp Ile Tyr Tyr Pro Gly Ser Val Lys
                50                  55                  60


        Gly Arg Phe Thr Ile Ser Arg Glu Asn Ala Lys Asn Ser Leu Tyr Leu
        65                  70                  75                  80


        Gln Met Asn Ser Leu Arg Ala Gly Asp Thr Ala Val Tyr Tyr Cys Ala
                        85                  90                  95


        Arg Gly Arg Tyr Ser Gly Ser Gly Ser Tyr Tyr Asn Asp Trp Phe Asp
                    100                 105                 110


        Pro Trp Gly Gln Gly Thr Leu Val Thr Val Ser Ser
                    115                 120
```

<210> 5
<211> 107
<212> PRT
<213> Homo sapiens

<400> 5

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Gly Ile Ser Ser Trp
            20                  25                  30

Leu Ala Trp Tyr Gln Gln Lys Pro Glu Lys Ala Pro Lys Ser Leu Ile
            35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr His Ser Tyr Pro Leu
                85                  90                  95

    Thr Phe Gly Gly Gly Thr Lys Val Glu Ile Lys
                100                 105
```

<210> 6
<211> 128
<212> PRT
<213> Homo sapiens

<400> 6

```
Gln Val Gln Leu Val Glu Ser Gly Gly Gly Val Val Gln Pro Gly Arg
1               5               10              15
```

```
Ser Leu Arg Leu Ser Cys Ala Ala Ser Gly Phe Thr Phe Ser Ser Tyr
            20              25              30
```

```
Gly Met His Trp Val Arg Gln Ala Pro Gly Glu Gly Leu Glu Trp Val
        35              40              45
```

```
Ala Val Ile Trp Tyr Asp Gly Thr Phe Lys Tyr Tyr Ala Asp Ser Val
    50              55              60
```

```
Lys Gly Arg Phe Thr Ile Ser Arg Asp Asn Ser Lys Asn Thr Leu Ser
65              70              75              80
```

```
Leu Leu Met Asn Ser Leu Arg Ala Glu Asp Thr Ala Val Tyr Tyr Cys
            85              90              95
```

```
Thr Arg Gly Gly Tyr Tyr Gly Ser Gly Ser Ser Phe Asp Tyr Tyr Tyr
        100             105             110
```

```
Tyr Gly Met Asp Val Trp Gly Gln Gly Thr Thr Val Thr Val Ser Ser
        115             120             125
```

<210> 7
<211> 107
<212> PRT
<213> Homo sapiens

<400> 7

```
Asp Ile Gln Met Thr Gln Ser Pro Ser Ser Leu Ser Ala Ser Val Gly
1               5               10              15
```

```
Asp Arg Val Thr Ile Thr Cys Arg Ala Ser Gln Gly Ile Ser Ser Trp
            20              25              30
```

```
Leu Ala Trp Tyr Gln Gln Lys Pro Glu Lys Ala Pro Lys Ser Leu Ile
        35              40              45
```

Tyr Ala Ala Ser Ser Leu Gln Ser Gly Val Pro Ser Arg Phe Ser Gly
        50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr His Ser Tyr Pro Leu
                85                  90                  95

Thr Phe Gly Gly Gly Thr Lys Val Glu Ile Lys
            100                 105

<210> 8
<211> 117
<212> PRT
<213> Homo sapiens

<400> 8

Gln Val Gln Leu Val Gln Ser Gly Ala Glu Val Lys Lys Pro Gly Ala
1                   5                   10                  15

Ser Val Lys Val Ser Cys Lys Val Ser Gly Tyr Thr Leu Thr Glu Leu
            20                  25                  30

Ser Met His Trp Val Arg Gln Ala Pro Gly Lys Gly Leu Glu Trp Met
        35                  40                  45

Gly Gly Phe Asp Pro Glu Asp Gly Glu Thr Ile Tyr Ala Gln Lys Phe
        50                  55                  60

Gln Gly Arg Val Thr Met Thr Glu Asp Thr Ser Thr Asp Thr Ala Tyr
65                  70                  75                  80

Met Glu Leu Ser Ser Leu Arg Ser Glu Asp Thr Ala Val Tyr Tyr Cys
                85                  90                  95

Ala Thr Asp Asp Leu Asp Tyr Phe Asp Tyr Trp Gly Gln Gly Thr Gln
            100                 105                 110

Val Thr Val Ser Ser
            115

<210> 9
<211> 108
<212> PRT
<213> Homo sapiens

<400> 9

```
Glu Ile Val Leu Thr Gln Ser Pro Ala Thr Leu Ser Leu Ser Pro Gly
1               5               10              15

Glu Arg Ala Thr Leu Ser Cys Arg Ala Ser Gln Ser Val Ser Ser Tyr
            20              25              30

Leu Ala Trp Tyr Gln Gln Lys Pro Gly Gln Ala Pro Arg Leu Leu Ile
        35              40              45

Tyr Asp Ala Ser Asn Arg Ala Thr Gly Ile Pro Ala Arg Phe Ser Gly
    50              55              60

Ser Gly Ser Gly Thr Asp Phe Thr Leu Thr Ile Ser Ser Leu Glu Pro
65              70              75              80

Glu Asp Phe Ala Val Tyr Tyr Cys Gln Gln Arg Ser Asn Trp Pro Pro
            85              90              95

Val Thr Phe Gly Gln Gly Thr Arg Leu Glu Ile Lys
            100             105
```

<210> 10
<211> 117
<212> PRT
<213> Homo sapiens

<400> 10

```
Gln Val Gln Leu Val Gln Ser Gly Ala Glu Val Lys Lys Pro Gly Ala
1               5               10              15

Ser Val Lys Val Ser Cys Lys Val Ser Gly Tyr Thr Leu Thr Glu Leu
            20              25              30

Ser Met His Trp Val Arg Gln Ala Pro Gly Lys Gly Leu Glu Trp Met
        35              40              45

Gly Gly Phe Asp Pro Glu Asp Gly Glu Thr Ile Tyr Ala Gln Lys Phe
    50              55              60

Gln Gly Arg Val Thr Met Thr Glu Asp Thr Ser Thr Asp Thr Ala Tyr
65              70              75              80

Met Glu Leu Ser Ser Leu Arg Ser Glu Asp Thr Ala Val Tyr Tyr Cys
            85              90              95

Ala Thr Asp Asp Leu Asp Tyr Phe Asp Tyr Trp Gly Gln Gly Thr Gln
            100             105             110
```

16

```
Val Thr Val Ser Ser
          115
```

<210> 11
<211> 108
<212> PRT
<213> Homo sapiens

<400> 11

```
Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
            35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Trp Pro Pro
            85                  90                  95

Val Thr Phe Gly Gln Gly Thr Arg Leu Glu Ile Lys
            100                 105
```

<210> 12
<211> 128
<212> PRT
<213> Homo sapiens

<400> 12

```
Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Glu Gly Leu Glu Trp Val
            35                  40                  45

Ala Val Ile Trp Tyr Asp Gly Thr Phe Lys Tyr Tyr Ala Asp Ser Val
    50                  55                  60
```

Lys Gly Arg Phe Thr Ile Ser Arg Asp Asn Ser Lys Asn Thr Leu Ser
65              70          75                  80

Leu Leu Met Asn Ser Leu Arg Ala Glu Asp Thr Ala Val Tyr Tyr Cys
            85              90                  95

Thr Arg Gly Gly Tyr Tyr Gly Ser Gly Ser Ser Phe Asp Tyr Tyr Tyr
            100             105             110

Tyr Gly Met Asp Val Trp Gly Gln Gly Thr Thr Val Thr Val Ser Ser
        115             120             125

<210> 13
<211> 107
<212> PRT
<213> Homo sapiens

<400> 13

Asp Ile Gln Met Thr Gln Ser Pro Ser Ser Leu Ser Ala Ser Val Gly
1               5           10              15

Asp Arg Val Thr Ile Thr Cys Arg Ala Ser Gln Gly Ile Ser Ser Trp
        20              25              30

Leu Ala Trp Tyr Gln Gln Lys Pro Glu Lys Ala Pro Lys Ser Leu Ile
        35              40              45

Tyr Ala Ala Ser Ser Leu Gln Ser Gly Val Pro Ser Arg Phe Ser Gly
    50              55              60

Ser Gly Ser Gly Thr Asp Phe Thr Leu Thr Ile Ser Ser Leu Gln Pro
65              70              75              80

Glu Asp Phe Ala Thr Tyr Tyr Cys Gln Gln Tyr Asn Ser Tyr Pro Leu
            85              90              95

Thr Phe Gly Gly Gly Thr Lys Val Glu Ile Lys
        100             105

<210> 14
<211> 124
<212> PRT
<213> Homo sapiens

<400> 14

18

```
Glu Val Gln Leu Val Glu Ser Gly Gly Gly Leu Val Gln Pro Gly Gly
1               5               10              15

Ser Leu Arg Leu Ser Cys Ala Ala Ser Gly Phe Thr Phe Ser Ser Tyr
            20                  25                  30

Asp Met His Trp Val Arg Gln Ala Thr Gly Lys Gly Leu Glu Trp Val
        35                  40                  45

Ser Ala Ile Ser Thr Ala Gly Asp Thr Tyr Tyr Pro Gly Ser Val Lys
    50                  55                  60

Gly Arg Phe Thr Ile Ser Arg Glu Asn Ala Lys Asn Ser Leu Tyr Leu
65                  70                  75                  80

Gln Met Asn Ser Leu Arg Val Gly Asp Thr Ala Val Tyr Tyr Cys Ala
            85                  90                  95

Arg Gly Arg Phe Asp Gly Ser Gly Ser Tyr Tyr Asn Asp Trp Phe Asp
            100                 105                 110

Pro Trp Gly Gln Gly Thr Leu Val Thr Val Ser Ser
        115                 120
```

<210> 15
<211> 107
<212> PRT
<213> Homo sapiens

<400> 15
```

```
Ala Ile Gln Leu Thr Gln Ser Pro Ser Ser Leu Ser Ala Ser Val Gly
1               5               10              15

Asp Arg Val Thr Ile Thr Cys Arg Ala Ser Gln Gly Ile Ser Ser Trp
            20              25              30

Leu Ala Trp Tyr Gln Gln Lys Pro Glu Lys Ala Pro Lys Ser Leu Ile
        35              40              45

Tyr Ala Ala Ser Ser Leu Gln Ser Gly Val Pro Ser Arg Phe Ser Gly
    50              55              60

Ser Gly Ser Gly Thr Asp Phe Thr Leu Thr Ile Ser Ser Leu Gln Pro
65              70              75              80

Glu Asp Phe Ala Thr Tyr Tyr Cys Gln Gln Tyr Asn Ser Tyr Pro Leu
            85              90              95

Thr Phe Gly Gly Gly Thr Lys Val Glu Ile Lys
        100             105
```

<210> 16
<211> 124
<212> PRT
<213> Homo sapiens

<400> 16

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Asp Met His Trp Val Arg Gln Ala Thr Gly Lys Gly Leu Glu Trp Val
        35                  40                  45

Ser Ala Ile Ser Thr Ala Gly Asp Thr Tyr Tyr Pro Gly Ser Val Lys
    50                  55                  60

Gly Arg Phe Thr Ile Ser Arg Glu Asn Ala Lys Asn Ser Leu Tyr Leu
65                  70                  75                  80

Gln Met Asn Ser Leu Arg Val Gly Asp Thr Ala Val Tyr Tyr Cys Ala
            85                  90                  95

Arg Gly Arg Phe Asp Gly Ser Gly Ser Tyr Tyr Asn Asp Trp Phe Asp
            100                 105                 110

Pro Trp Gly Gln Gly Thr Leu Val Thr Val Ser Ser
        115                 120
```

<210> 17
<211> 107
<212> PRT
<213> Homo sapiens

<400> 17

```
Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80
```

```
        Glu Asp Phe Ala Val Tyr Tyr Cys Gln Gln Arg Tyr Asn Trp Pro Leu
                    85              90              95


        Thr Phe Gly Gly Gly Thr Lys Val Glu Ile Lys
                    100             105
```

<210> 18
<211> 124
<212> PRT
<213> Homo sapiens

<400> 18

```
        Glu Val Gln Leu Val Glu Ser Gly Gly Gly Leu Val Gln Pro Gly Gly
        1               5               10              15


        Ser Leu Arg Leu Ser Cys Ala Ala Ser Gly Phe Thr Phe Ser Ser Tyr
                    20              25              30


        Asp Met His Trp Val Arg Gln Ala Thr Gly Lys Gly Leu Glu Trp Val
                    35              40              45


        Ser Ala Ile Ser Thr Ala Gly Asp Thr Tyr Tyr Pro Gly Ser Val Lys
                50              55              60


        Gly Arg Phe Thr Ile Ser Arg Glu Asn Ala Lys Asn Ser Leu Tyr Leu
        65              70              75              80


        Gln Met Asn Ser Leu Arg Val Gly Asp Thr Ala Val Tyr Tyr Cys Ala
                    85              90              95


        Arg Gly Arg Phe Asp Gly Ser Gly Ser Tyr Tyr Asn Asp Trp Phe Asp
                    100             105             110


        Pro Trp Gly Gln Gly Thr Leu Val Thr Val Ser Ser
                    115             120
```

<210> 19
<211> 108
<212> PRT
<213> Homo sapiens

<400> 19

```
        Glu Ile Val Leu Thr Gln Ser Pro Gly Thr Leu Ser Leu Ser Pro Gly
        1               5               10              15


        Glu Arg Ala Thr Leu Ser Cys Arg Ala Ser Gln Ser Val Ser Ser Ser
                    20              25              30
```

```
      Tyr Leu Ala Trp Tyr Gln Gln Lys Pro Gly Gln Ala Pro Arg Leu Leu
              35                  40                  45


      Ile Tyr Gly Ala Ser Ser Arg Ala Thr Gly Ile Pro Asp Arg Phe Ser
              50                  55                  60


      Gly Ser Gly Ser Gly Thr Asp Phe Thr Leu Thr Ile Ser Arg Leu Glu
      65                  70                  75                  80


      Pro Glu Asp Phe Ala Val Tyr Tyr Cys Gln Gln Tyr Gly Ser Ser Pro
                      85                  90                  95


      Phe Thr Phe Gly Pro Gly Thr Lys Val Asp Ile Lys
                  100                 105
```

<210> 20
<211> 119
<212> PRT
<213> Homo sapiens

<400> 20

```
      Gln Val Gln Leu Val Glu Ser Gly Gly Gly Val Val Gln Pro Gly Arg
      1               5                   10                  15


      Ser Leu Arg Leu Ser Cys Ala Ala Thr Gly Phe Thr Phe Ser Ser Tyr
                  20                  25                  30


      Gly Met His Trp Val Arg Gln Ala Pro Gly Lys Gly Leu Glu Trp Val
              35                  40                  45


      Ala Val Ile Trp Tyr Asp Gly Ser Lys Lys Tyr Tyr Thr Asp Ser Val
              50                  55                  60


      Lys Gly Arg Phe Thr Ile Ser Arg Asp Asn Ser Lys Asn Thr Leu Tyr
      65                  70                  75                  80


      Leu Gln Met Asn Ser Leu Arg Ala Glu Asp Thr Ala Val Tyr Tyr Cys
                      85                  90                  95


      Ala Arg Asp Gln Asn Trp Ile Asp Val Phe Asp Ile Trp Gly Gln Gly
                  100                 105                 110


      Thr Met Val Thr Val Ser Ser
                  115
```

<210> 21
<211> 107
<212> PRT

<213> Homo sapiens

<400> 21

```
Ala Ile Gln Leu Thr Gln Ser Pro Ser Ser Leu Ser Ala Ser Val Gly
1               5                   10                  15

Asp Arg Val Thr Ile Thr Cys Arg Ala Ser Gln Gly Ile Ser Ser Ala
            20                  25                  30

Leu Ala Trp Tyr Gln Gln Lys Pro Gly Lys Ala Pro Lys Leu Leu Ile
            35                  40                  45

Tyr Asp Ala Ser Ser Leu Glu Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Phe Asn Ser Tyr Pro Tyr
                85                  90                  95

Thr Phe Gly Gln Gly Thr Lys Leu Glu Ile Lys
            100                 105
```

<210> 22
<211> 124
<212> PRT
<213> Homo sapiens

<400> 22

```
Gln Pro Gln Leu Val Gln Ser Gly Ala Glu Val Lys Lys Pro Gly Ala
1               5                   10                  15

Ser Val Lys Val Ser Cys Lys Val Ser Gly Asn Thr Leu Thr Glu Leu
            20                  25                  30

Ser Met His Trp Val Arg Gln Ala Pro Gly Lys Gly Leu Glu Trp Met
            35                  40                  45

Gly Gly Phe Asp Pro Glu Asn Gly Glu Ala Ile Tyr Ala Gln Lys Phe
    50                  55                  60

Gln Gly Arg Val Thr Met Thr Ala Asp Thr Ser Thr Asp Thr Ala Tyr
65                  70                  75                  80

Met Asp Leu Ser Ser Leu Arg Ser Glu Asp Thr Ala Val Tyr Tyr Cys
                85                  90                  95
```

```
Ala Thr Asp Leu Ala Gly Gly Ser Asp Phe Tyr Tyr Tyr Gly Leu Asp
            100             105             110

Val Trp Gly Gln Gly Thr Thr Val Thr Val Ser Ser
        115             120
```

<210> 23
<211> 107
<212> PRT
<213> Homo sapiens

<400> 23

```
Arg Thr Val Ala Ala Pro Ser Val Phe Ile Phe Pro Pro Ser Asp Glu
1               5               10              15

Gln Leu Lys Ser Gly Thr Ala Ser Val Val Cys Leu Leu Asn Asn Phe
            20              25              30

Tyr Pro Arg Glu Ala Lys Val Gln Trp Lys Val Asp Asn Ala Leu Gln
            35              40              45

Ser Gly Asn Ser Gln Glu Ser Val Thr Glu Gln Asp Ser Lys Asp Ser
        50              55              60

Thr Tyr Ser Leu Ser Ser Thr Leu Thr Leu Ser Lys Ala Asp Tyr Glu
65              70              75              80

Lys His Lys Val Tyr Ala Cys Glu Val Thr His Gln Gly Leu Ser Ser
            85              90              95

Pro Val Thr Lys Ser Phe Asn Arg Gly Glu Cys
            100             105
```

<210> 24
<211> 330
<212> PRT
<213> Homo sapiens

<400> 24

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
```

```
              50                        55                        60
```

Leu Ser Ser Val Val Thr Val Pro Ser Ser Ser Leu Gly Thr Gln Thr
65              70              75              80

Tyr Ile Cys Asn Val Asn His Lys Pro Ser Asn Thr Lys Val Asp Lys
                85              90              95

Lys Val Glu Pro Lys Ser Cys Asp Lys Thr His Thr Cys Pro Pro Cys
            100             105             110

Pro Ala Pro Glu Leu Leu Gly Gly Pro Ser Val Phe Leu Phe Pro Pro
        115             120             125

Lys Pro Lys Asp Thr Leu Met Ile Ser Arg Thr Pro Glu Val Thr Cys
        130             135             140

Val Val Val Asp Val Ser His Glu Asp Pro Glu Val Lys Phe Asn Trp
145             150             155             160

Tyr Val Asp Gly Val Glu Val His Asn Ala Lys Thr Lys Pro Arg Glu
                165             170             175

Glu Gln Tyr Asn Ser Thr Tyr Arg Val Val Ser Val Leu Thr Val Leu
            180             185             190

His Gln Asp Trp Leu Asn Gly Lys Glu Tyr Lys Cys Lys Val Ser Asn
        195             200             205

Lys Ala Leu Pro Ala Pro Ile Glu Lys Thr Ile Ser Lys Ala Lys Gly
        210             215             220

Gln Pro Arg Glu Pro Gln Val Tyr Thr Leu Pro Pro Ser Arg Asp Glu
225             230             235             240

Leu Thr Lys Asn Gln Val Ser Leu Thr Cys Leu Val Lys Gly Phe Tyr
            245             250             255

Pro Ser Asp Ile Ala Val Glu Trp Glu Ser Asn Gly Gln Pro Glu Asn
            260             265             270

Asn Tyr Lys Thr Thr Pro Pro Val Leu Asp Ser Asp Gly Ser Phe Phe
        275             280             285

Leu Tyr Ser Lys Leu Thr Val Asp Lys Ser Arg Trp Gln Gln Gly Asn
    290             295             300

```
Val Phe Ser Cys Ser Val Met His Glu Ala Leu His Asn His Tyr Thr
305             310             315             320
```

```
Gln Lys Ser Leu Ser Leu Ser Pro Gly Lys
            325             330
```

<210> 25
<211> 329
<212> PRT
<213> Homo sapiens


<400> 25

```
Ala Ser Thr Lys Gly Pro Ser Val Phe Pro Leu Ala Pro Ser Ser Lys
1               5               10              15
```

```
Ser Thr Ser Gly Gly Thr Ala Ala Leu Gly Cys Leu Val Lys Asp Tyr
            20              25              30
```

```
Phe Pro Glu Pro Val Thr Val Ser Trp Asn Ser Gly Ala Leu Thr Ser
        35              40              45
```

```
Gly Val His Thr Phe Pro Ala Val Leu Gln Ser Ser Gly Leu Tyr Ser
    50              55              60
```

```
Leu Ser Ser Val Val Thr Val Pro Ser Ser Ser Leu Gly Thr Gln Thr
65              70              75              80
```

```
Tyr Ile Cys Asn Val Asn His Lys Pro Ser Asn Thr Lys Val Asp Lys
            85              90              95
```

```
Lys Val Glu Pro Lys Ser Cys Asp Lys Thr His Thr Cys Pro Pro Cys
            100             105             110
```

```
Pro Ala Pro Pro Val Ala Gly Pro Ser Val Phe Leu Phe Pro Pro Lys
    115             120             125
```

```
Pro Lys Asp Thr Leu Met Ile Ser Arg Thr Pro Glu Val Thr Cys Val
    130             135             140
```

```
Val Val Asp Val Ser His Glu Asp Pro Glu Val Lys Phe Asn Trp Tyr
145             150             155             160
```

```
Val Asp Gly Val Glu Val His Asn Ala Lys Thr Lys Pro Arg Glu Glu
            165             170             175
```

```
Gln Tyr Asn Ser Thr Tyr Arg Val Val Ser Val Leu Thr Val Leu His
            180             185             190
```

EP 2 328 559 B1

```
Gln Asp Trp Leu Asn Gly Lys Glu Tyr Lys Cys Lys Val Ser Asn Lys
        195                 200             205

Gly Leu Pro Ser Ser Ile Glu Lys Thr Ile Ser Lys Ala Lys Gly Gln
    210                 215                 220

Pro Arg Glu Pro Gln Val Tyr Thr Leu Pro Pro Ser Arg Asp Glu Leu
225                 230                 235                 240

Thr Lys Asn Gln Val Ser Leu Thr Cys Leu Val Lys Gly Phe Tyr Pro
                245                 250                 255

Ser Asp Ile Ala Val Glu Trp Glu Ser Asn Gly Gln Pro Glu Asn Asn
            260                 265                 270

Tyr Lys Thr Thr Pro Pro Val Leu Asp Ser Asp Gly Ser Phe Phe Leu
        275                 280                 285

Tyr Ser Lys Leu Thr Val Asp Lys Ser Arg Trp Gln Gln Gly Asn Val
    290                 295                 300

Phe Ser Cys Ser Val Met His Glu Ala Leu His Asn His Tyr Thr Gln
305                 310                 315                 320

Lys Ser Leu Ser Leu Ser Pro Gly Lys
                325
```

<210> 26
<211> 330
<212> PRT
<213> Homo sapiens

<400> 26

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80
```

29

Tyr Ile Cys Asn Val Asn His Lys Pro Ser Asn Thr Lys Val Asp Lys
                85                    90                    95

Lys Val Glu Pro Lys Ser Cys Asp Lys Thr His Thr Cys Pro Pro Cys
            100                   105                   110

Pro Ala Pro Glu Ala Ala Gly Gly Pro Ser Val Phe Leu Phe Pro Pro
            115                   120                   125

Lys Pro Lys Asp Thr Leu Met Ile Ser Arg Thr Pro Glu Val Thr Cys
    130                   135                   140

Val Val Val Asp Val Ser His Glu Asp Pro Glu Val Lys Phe Asn Trp
145                   150                   155                   160

Tyr Val Asp Gly Val Glu Val His Asn Ala Lys Thr Lys Pro Arg Glu
            165                   170                   175

Glu Gln Tyr Asn Ser Thr Tyr Arg Val Val Ser Val Leu Thr Val Leu
            180                   185                   190

His Gln Asp Trp Leu Asn Gly Lys Glu Tyr Lys Cys Lys Val Ser Asn
            195                   200                   205

Lys Ala Leu Pro Ala Pro Ile Glu Lys Thr Ile Ser Lys Ala Lys Gly
    210                   215                   220

Gln Pro Arg Glu Pro Gln Val Tyr Thr Leu Pro Pro Ser Arg Asp Glu
225                   230                   235                   240

Leu Thr Lys Asn Gln Val Ser Leu Thr Cys Leu Val Lys Gly Phe Tyr
            245                   250                   255

Pro Ser Asp Ile Ala Val Glu Trp Glu Ser Asn Gly Gln Pro Glu Asn
            260                   265                   270

Asn Tyr Lys Thr Thr Pro Pro Val Leu Asp Ser Asp Gly Ser Phe Phe
    275                   280                   285

Leu Tyr Ser Lys Leu Thr Val Asp Lys Ser Arg Trp Gln Gln Gly Asn
    290                   295                   300

Val Phe Ser Cys Ser Val Met His Glu Ala Leu His Asn His Tyr Thr
305                   310                   315                   320

Gln Lys Ser Leu Ser Leu Ser Pro Gly Lys
            325                   330

<210> 27
<211> 327
<212> PRT
<213> Homo sapiens

<400> 27

```
Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Ser Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220
```

```
Glu Pro Gln Val Tyr Thr Leu Pro Pro Ser Gln Glu Glu Met Thr Lys
225                 230             235                 240

Asn Gln Val Ser Leu Thr Cys Leu Val Lys Gly Phe Tyr Pro Ser Asp
                245             250                 255

Ile Ala Val Glu Trp Glu Ser Asn Gly Gln Pro Glu Asn Asn Tyr Lys
                260             265                 270

Thr Thr Pro Pro Val Leu Asp Ser Asp Gly Ser Phe Phe Leu Tyr Ser
            275             280                 285

Arg Leu Thr Val Asp Lys Ser Arg Trp Gln Glu Gly Asn Val Phe Ser
        290             295             300

Cys Ser Val Met His Glu Ala Leu His Asn His Tyr Thr Gln Lys Ser
305                 310             315                 320

Leu Ser Leu Ser Leu Gly Lys
                325
```

<210> 28
<211> 327
<212> PRT
<213> Homo sapiens

<400> 28

```
Ala Ser Thr Lys Gly Pro Ser Val Phe Pro Leu Ala Pro Cys Ser Arg
1               5               10                  15

Ser Thr Ser Glu Ser Thr Ala Ala Leu Gly Cys Leu Val Lys Asp Tyr
                20              25                  30

Phe Pro Glu Pro Val Thr Val Ser Trp Asn Ser Gly Ala Leu Thr Ser
            35              40                  45

Gly Val His Thr Phe Pro Ala Val Leu Gln Ser Ser Gly Leu Tyr Ser
        50              55                  60

Leu Ser Ser Val Val Thr Val Pro Ser Ser Ser Leu Gly Thr Lys Thr
65              70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Pro Cys Pro Ala Pro
            100                 105                 110
```

Glu Phe Glu Gly Gly Pro Ser Val Phe Leu Phe Pro Pro Lys Pro Lys
115 120 125

Asp Thr Leu Met Ile Ser Arg Thr Pro Glu Val Thr Cys Val Val Val
130 135 140

Asp Val Ser Gln Glu Asp Pro Glu Val Gln Phe Asn Trp Tyr Val Asp
145 150 155 160

Gly Val Glu Val His Asn Ala Lys Thr Lys Pro Arg Glu Glu Gln Phe
165 170 175

Asn Ser Thr Tyr Arg Val Val Ser Val Leu Thr Val Leu His Gln Asp
180 185 190

Trp Leu Asn Gly Lys Glu Tyr Lys Cys Lys Val Ser Asn Lys Gly Leu
195 200 205

Pro Ser Ser Ile Glu Lys Thr Ile Ser Lys Ala Lys Gly Gln Pro Arg
210 215 220

Glu Pro Gln Val Tyr Thr Leu Pro Pro Ser Gln Glu Glu Met Thr Lys
225 230 235 240

Asn Gln Val Ser Leu Thr Cys Leu Val Lys Gly Phe Tyr Pro Ser Asp
245 250 255

Ile Ala Val Glu Trp Glu Ser Asn Gly Gln Pro Glu Asn Asn Tyr Lys
260 265 270

Thr Thr Pro Pro Val Leu Asp Ser Asp Gly Ser Phe Phe Leu Tyr Ser
275 280 285

Arg Leu Thr Val Asp Lys Ser Arg Trp Gln Glu Gly Asn Val Phe Ser
290 295 300

Cys Ser Val Met His Glu Ala Leu His Asn His Tyr Thr Gln Lys Ser
305 310 315 320

Leu Ser Leu Ser Leu Gly Lys
325

## Claims

1. A pharmaceutical formulation comprising:

   1 to 50 mg/mL huMAb-P-selectin;

20 mM of L-histidine HCl buffer;
0.02% of polysorbate 20;
240 mM of trehalose;
at pH 6.0,
wherein huMAb-P-selectin is an antibody against P-selectin comprising the light chain variable domain defined by amino acid sequence SEQ ID NO:3 and the heavy chain variable domain defined by SEQ ID NO:4.

2. The formulation according to claim 1, which is a liquid formulation or a lyophilized formulation or a liquid formulation reconstituted from a lyophilized formulation.

3. A formulation according to claims 1 or 2 for use in treating asthma or allergy.


**Patentansprüche**

1. Pharmazeutische Formulierung, die umfasst:

1 bis 50 mg/ml hu-MAb-P-Selektin;
20 mM L-Histidin HCl-Puffer;
0,02% Polysorbat 20;
240 mM Trehalose;
bei pH 6,0,
wobei huMAb-P-Selektin ein Antikörper gegen P-Selektin ist, umfassend die variable Domäne der leichten Kette, die definiert ist durch die Aminosäuresequenz SEQ ID NO:3, und die variable Domäne der schweren Kette, die definiert ist durch SEQ ID NO:4.

2. Formulierung nach Anspruch 1, die eine flüssige Formulierung ist oder eine lyophilisierte Formulierung oder eine aus einer lyophilisierten Formulierung rekonstituierte flüssige Formulierung.

3. Formulierung nach Anspruch 1 oder 2 zur Verwendung in der Behandlung von Asthma oder Allergie.


**Revendications**

1. Formulation pharmaceutique comprenant :

1 à 50 mg/ml de huMAb-P-sélectine ;
20 mM de tampon au L-histidine-HCl ;
0,02 % de polysorbate 20 ;
240 mM de tréhalose ;
à pH 6,0,
dans laquelle la huMAb-P-sélectine est un anticorps contre la P-sélectine comprenant le domaine variable de chaîne légère défini par la séquence d'amino-acides SEQ ID N°:3 et le domaine variable de chaîne lourde défini par la SEQ ID N°:4.

2. Formulation suivant la revendication 1, qui est une formulation liquide ou une formulation lyophilisée ou bien une formulation liquide reconstituée à partir d'une formulation lyophilisée.

3. Formulation suivant la revendication 1 ou 2, pour une utilisation dans le traitement de l'asthme ou d'une allergie.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4783399 A
- WO 9306863 A
- WO 9321956 A
- WO 2005100402 A
- US 20030138417 A
- WO 2006072564 A

### Non-patent literature cited in the description

- **GENG et al.** *J. Biol. Chem.,* 1991, vol. 266, 22313-22318
- **KABAT et al.** Sequences of Proteins of Immunological Interest. National Institutes of Health, 1991
- **JOHNSON ; WU.** *Nucleic Acids Res.,* 2000, vol. 28, 214-218
- **ROMO et al.** *J Exp Med,* 1999, vol. 190, 803
- **RIECHMANN et al.** *Nature,* 1988, vol. 332, 323-327
- **QUEEN et al.** *Proc. Natl. Acad. Sci. USA,* 1989, vol. 86, 10029-10033
- **CLELAND et al.** *Crit Rev Ther Drug Carrier Syst,* 1993, vol. 10 (4), 307-77
- **WANG.** *Int J Pharm,* 1999, vol. 185 (2), 129-88
- **WANG.** *Int J Pharm,* 2000, vol. 203 (1-2), 1-60
- **CHI et al.** *Pharm Res,* 2003, vol. 20 (9), 1325-36